# EUROPEAN PATENT SPECIFICATION

(11) **EP 2 641 962 B1**
(45) Date of publication and mention of the grant of the patent: **14.03.2018**
(21) Application number: 11841638.7
(22) Date of filing: 18.11.2011
(51) Int. Cl.: C12N 1/16, C12P 7/06, C12G 3/02, C12R 1/84

(54) **PICHIA KLUYVERI STRAIN AND USES THEREOF**
PICHIA-KLUYVERI-STAMM UND DESSEN VERWENDUNGEN
SOUCHE DE PICHIA KLUYVERI ET SES APPLICATIONS

(30) Priority: 19.11.2010 ES 201031705 P
(43) Date of publication of application: 25.09.2013
(73) Proprietor: Universidad Pablo De Olavide, 41013 Sevilla (ES); Grupo Hespérides Biotech, S.L., 41013 Sevilla (ES); Ripened Ventures, Inc., Emeryville, CA 94608 (US)
(72) Inventor: BARRERA GARCÍA, Juan Alberto, E-41013 Sevilla (ES); CORDÓN TOLEDANO, Juan Diego, E-41013 Sevilla (ES); CORONEL DOMÍNGUEZ, Antonio Jesús, E-41013 Sevilla (ES); FERNÁNDEZ ZABALA, Cristóbal, E-41013 Sevilla (ES); SANTOS OCAÑA, Carlos, E-41013 Sevilla (ES)
(74) Representative: ABG Patentes, S.L.
(86) International application number: PCT/ES2011/070793
(87) International publication number: WO 2012/066176

(56) References cited:
- WO-A1-2009/110807
- WO-A2-2007/141420
- ES-A1- 2 164 565
- ES-A1- 2 222 091
- ES-A1- 2 222 091
- FR-A1- 2 657 878
- US-A1- 2002 172 738
- US-A1- 2002 172 738
- ARIAS COVADONGA R ET AL: "Yeast species associated with orange juice: Evaluation of different identification methods", APPLIED AND ENVIRONMENTAL MICROBIOLOGY, vol. 68, no. 4, April 2002 (2002-04), pages 1955-1961, XP002722436, ISSN: 0099-2240

## Description

### FIELD OF THE INVENTION

The invention relates to a strain of *Pichia kluyveri* that is able to naturally ferment orange juice and the use thereof to produce a drink with low alcohol content.

### BACKGROUND OF THE INVENTION

Alcoholic fermentation is perhaps the first biotechnological process undertaken by humans. Although it was initially used as a means of preserving fruit, over the years it subsequently became more widely used as a method for obtaining alcoholic drinks such as wines. Drinks obtained by fermentation of fruit juices are generally supposed to refer to the wine obtained from grape juice.

Most wine is obtained from grapes and, unless another source is specified, the word wine refers to the product resulting from the fermentation of grape juice (Brown et al., 1989. 1st ed. Blackwell Scientific Publications, Oxford, England). The production of wine from fruits other than grape is very popular in many countries in northern Europe, especially Poland, Russia and Germany, where climate conditions prevent winegrowing. In the UK, only a small quantity of wine from other fruits is produced commercially, although home-based winemaking is very popular.

The yeast populations present in the ecosystem from which the fruits are obtained have traditionally been used to ferment the must from these fruits. The fermentation process results in an evolution of the microbial populations in which one population replaces another, thus meaning that the chemical composition of the fermented medium is continually changing. In the specific case of orange fermentation, the process presents one key difference and an additional problem as regards musts from other fruits. Said difference lies in the fact that *Lactobacillus* displaces all other microorganisms during the initial stages of fermentation, thereby leading to a build-up of lactic acid and resulting in a product with an unpleasant taste and smell. The spontaneous fermentation of orange juice does not therefore lead to a product with the organoleptic properties obtained when using musts such as grape, apple, barley, etc.

The most abundant yeasts present in orange juice are *Hanseniaspora uvarum (27%), Hanseniaspora occidentalis* (15%), *Pichia kluyveri* (9%), *Candida intermedia* (7%) and *Candida parapsilosis* (6%) (Arias et al. Applied and Environmental Microbiology, 2002, 68:1955-1961).

Specifically, *Pichia* is a genus of the family *Saccharomycetaceae* with spherical, elliptical or oblong cells. There are 91 known species in the genus *Pichia* and three varieties of the specific species *P. kluyveri,* namely *eremophila, cephalocereana* and *kluyveri* (Phaff, Starmer and Tredick-Kline, 1987, Studies Mycol 30:412). Members of the genus *Pichia* are unable to either ferment or assimilate lactose. The fermentation of other carbohydrates varies with the species. Specifically, *Pichia kluyveri* is able to ferment glucose but not galactose, sucrose, maltose, raffinose or trehalose. Species of the genus *Pichia,* such as *P. kluyveri* and *P. fermentans,* are responsible for the deterioration of fruits such as pears, tomatoes, oranges, etc.

There are some literature precedents from many years ago for the production of fermented citrus juices, such as those of Hendrickson and Kesterson (1965) (Agric. Exp. Sta. Bull. 698: 64-66), which cites the work of von Loesecke (1936), which describes various products derived from the manufacture of citrus fruits, including grapefruit and orange wines. In the latter case, it is reported that, although the juice from these citrus fruits may be converted into wine with a pleasant smell and taste, additional sugar must be added and the manufacturing conditions must be carefully controlled. Braverman (Composición y tecnologia química. Madrid: Aguilar. 1952) mentions and describes the steps required to obtain "sour wines", which are defined as products obtained by fermentation of juices obtained from mature fruits from less acidic varieties, spiked with sugar, using wine yeasts to obtain fuller-bodied and less acidic wines. The most recent scientific understanding regarding the preparation of citric wines comes from Turkey and other Asian countries (China, Japan and Korea). Amongst others, the studies concerning the production of orange wine published by Arici-Oe et al. (1994) (Gida 19, 2:113-117) in China, the isolation and identification of yeasts for the production of orange wine by Young-Hwan et al. (1997) (Korean J. Food Sci. Technol. 29, 3: 588-594) in Korea and basic studies into techniques for orange wine production by You-Young and Guo-Qing (2000) (J. Zhejiang Univ. Agric. Life Sci. 26, 5: 513-515) in China can be cited.

Moreover, a procedure for obtaining such wine from an orange juice by-product involving fermentation with alcohol-producing yeasts has been described (ES 2164565). Specifically, the yeasts used belong to the genera *Hanseniospora* and *Saccharomyces.* Moreover, a procedure for fermenting natural orange juice in which two yeast strains are added sequentially to the orange juice has also been reported. Specifically, said yeasts are *Pichia fermentans* CECT 11773 and *Saccharomyces cerevisiae.*

Additionally, a fermentation process for producing alcoholic sweetened orange beverages has been described in US 2002/0172738A1. In this case, the fermentation is caused by the incorporation of mineral salts and a combination of three different yeasts to the reaction, being said yeasts *Saccharomyces cerevisiae, Saccharomyces carlsbergensis and* Epernay. This procedure yields a product with a relatively high ethanol content which needs sweetening.

Consequently, although various microorganisms that are able to ferment orange juice have been described, there remains a need to develop a procedure that results in a drink with better organoleptic properties and a lower alcohol content.

### SUMMARY OF THE INVENTION

The inventors have identified a strain of yeast from the species *Pichia kluyveri* that is able to ferment orange juice to give an orange juice-derived drink with excellent organoleptic properties for consumption and a low alcohol content of between 0.1% and 3.4% (v/v), typically around 2.5%. A culture of said *P. kluyveri* isolate was deposited in the Spanish Type Culture Collection (CECT) on 12 May 2010 with accession number CECT 13055.

The presence of an appropriate concentration of pulp in the source orange juice is an advantage as said pulp represents an oxygen reservoir for the yeast, which is required in the initial stages of fermentation, and also provides large quantities of nutrients. Moreover, the ability of the yeast strain of the invention to only ferment the reducing sugars present in the juice leads to a final product containing non-reducing sugars, such as sucrose, thus providing said product with its pleasant taste.

Consequently, one aspect of the invention relates to a microorganism from the species *Pichia kluyveri* deposited in the Spanish Type Culture Collection (CECT) with accession number 13055 that is able to ferment glucose and/or fructose, or a mutant of said microorganism that maintains said ability. A culture comprising said microorganism constitute an additional aspect of this invention.

In another aspect, the invention relates to a culture medium comprising the microorganism of the invention, further comprising 25% (v/v) orange juice and 1% (w/v) of a carbon source.

In a further aspect, the invention relates to the use of said microorganism or said culture to perform alcoholic fermentation.

In another aspect, the invention relates to a growth method for said microorganism or culture comprising inoculation of said microorganism in a medium comprising orange juice.

In a further aspect, the invention relates to a method for obtaining an orange juice by-product comprising inoculation of said microorganism or a culture thereof into a culture medium comprising orange juice under conditions that allow fermentation of the reducing sugars in said orange juice.

In another aspect, the invention relates to an orange juice by-product comprising said microorganism, containing an ethanol concentration of between 0.1% and 3.4% (v/v) that is obtained using a method comprising inoculation of said microorganism into a culture medium comprising orange juice under conditions that allow fermentation of the reducing sugars present in said orange juice.

### BRIEF DESCRIPTION OF THE FIGURES

**Figure 1** shows the growth evolution for yeast strain 4N187 (CECT 13055) in different media, showing the growth time in hours versus the optical density measured at 660 nm. YPD: glucose-enriched medium; YPE: ethanol-enriched medium; YPG: glycerol-enriched medium; 25% juice: medium comprising 25% orange juice in water; sumo: 100% orange juice.
**Figure 2** shows the evolution of the concentration of total sugars consumed (g/1) during fermentation for yeast strain 4N187 (CECT 13055).
**Figure 3** shows the evolution of the concentration of reducing sugars available in the culture medium (g/1) during fermentation for yeast strain 4N187 (CECT 13055).
**Figure 4** shows the evolution of the ethanol concentration (% v/v) with fermentation time for yeast strain 4N187 (CECT 13055).
**Figure 5** shows the evolution of the degrees Brix (°Bx) with fermentation time for yeast strain 4N187 (CECT 13055).
**Figure 6** shows the evolution of the concentration of reducing sugars available in the culture medium (g/1) during the first five days of fermentation for the following yeast strains: *P. kluyveri* 4N187 (CECT 13055), *P. kluyveri* 4N179, *P. fermentans, S. cerevisiae, S. carlbengensis* and *S. cerevisiae* var. *bayanus.*
**Figure 7** shows the evolution of the concentration of non-reducing sugars, specifically sucrose, available in the culture medium (g/1) during fermentation for the following different yeasts from the genus *Pichia*: *P. kluyveri* 4N187 (CECT 13055), *P. kluyveri* 4N179 and *P. fermentans.*
**Figure 8** shows the evolution of the concentration of non-reducing sugars, specifically sucrose, available in the culture medium (g/1) during fermentation for the following different yeasts from the genus *Saccharomyces: S. cerevisiae, S. carlbengensis* and *S. cerevisiae* var. *bayanus.*
**Figure 9** shows the evolution of the concentration of reducing sugars available in the culture medium (g/1) during fermentation for the following yeast strains: P. *kluyveri* 4N187 (CECT 13055), *P. kluyveri* 4N179, *P. fermentans, S. cerevisiae, S. carlbengensis* and *S. cerevisiae* var. *bayanus.*
**Figure 10** shows the evolution of the ethanol concentration (% v/v) generated during fermentation for the following yeast strains: *P. kluyveri* 4N187 (CECT 13055), *P. kluyveri* 4N179, *P. fermentans, S. cerevisiae, S. carlbengensis* and *S. cerevisiae* var. *bayanus.*
**Figure 11** shows the evolution of the ethanol concentration (% v/v) generated during stirred fermentation for the following different strains of the genus *Pichia*: *P. kluyveri* 4N187 (CECT 13055), *P. kluyveri* 4N179 and *P.fermentans.*
**Figure 12** shows the evolution of the ethanol concentration (% v/v) generated during fermentation for the following different strains of the genus *Saccharomyces: S. cerevisiae, S. carlbengensis* and *S. cerevisiae* var. *bayanus.*
**Figure 13** shows the evolution of the ethanol concentration (% v/v) generated during fermentation at-rest for the following different strains of the genus *Pichia*: *P. kluyveri* 4N187 (CECT 13055), *P. kluyveri* 4N179 and *P.fermentans.*

### DETAILED DESCRIPTION OF THE INVENTION

In general, the invention relates to a microorganism that is able to ferment reducing sugars, specifically glucose and/or fructose, as described in example 3, in which it can be seen that, after fermentation, the concentration of reducing sugars is essentially zero.

### Microorganism of the invention

In one aspect, the invention relates to a microorganism, hereinafter "microorganism of the invention", from the species *Pichia kluyveri* deposited in the Spanish Type Culture Collection (CECT) with accession number 13055 that is able to ferment glucose and/or fructose, or a mutant of said microorganism that maintains said ability.

As used herein, the expression "able to ferment glucose and/or fructose" means that the microorganism of the invention is able to ferment said reducing sugars. The term "fermentation" refers to an incomplete catabolic and fully anaerobic oxidation process, the final product of which is an organic compound. Specifically, the microorganism of the invention is able to ferment reducing sugars, such as glucose and/or fructose, by an alcoholic fermentation process in which the microorganism of the invention processes said carbohydrates to obtain ethanol, gaseous carbon dioxide (CO₂) and molecules of adenosine triphosphate (ATP), with said molecules being consumed by the microorganism as part of the anaerobic cellular energy metabolism thereof.

As used in the present invention, the term "reducing sugars (or carbohydrates)" relates to those sugars possessing an intact carbonyl group (functional group) and which can react with other molecules via said group. Said reducing sugars alter proteins by way of a non-enzymatic glycosylation reaction also known as the "Maillard reaction" or glycation. Examples of reducing sugars include, but are not limited to, both mono- and polysaccharides such as glucose, fructose, glyceraldehyde, galactose, lactose and maltose. Specifically, the microorganism of the invention is able to ferment glucose and/or fructose.

In a particular embodiment, as well as being able to ferment reducing sugars, the microorganism of the invention, does not have (lacks) the ability to ferment non-reducing sugars. As can be seen in example 3, *P. kluyveri* strain 4N187 (CECT 13055) is unable to ferment non-reducing sugars, for example sucrose, thereby resulting in an end product to which no sweeteners need be added due to the sucrose concentration present.

In a particular embodiment, the microorganism of the invention is a mutant of said *Pichia kluyveri* strain (CECT 13055) that maintains the ability to ferment glucose and/or fructose. As used in the present invention, the term "mutant" includes any individual or organism resulting from a genetic mutation or modification in the genome of said organism. Said organism conserves the ability to ferment reducing sugars, specifically to ferment glucose and/or fructose.

The ability of a microorganism or a mutant of said microorganism to ferment reducing sugars, specifically glucose and/or fructose, can be determined by conventional methods, for example, growing said microorganism is a medium comprising said reducing sugars (glucose and/or fructose) and checking whether said reducing sugars have disappeared from the medium at the end of the fermentation or whether the initial quantities of said sugars have decreased. Appropriate methods for assaying reducing sugars include the 3,5-dinitrosalicylic acid (DNS) colorimetric method (Miller, G. L. 1959. Anal. Chem, 31; 426-428), use of the Fehling reagent, chromatographic methods, etc. In a particular embodiment, the reducing sugars are assayed using the 3,5-dinitrosalicylic acid (DNS) colorimetric method.

The microorganism of the invention can be cultured in any medium suitable for yeasts. As a person skilled in the art will understand, said culture medium comprises the nutrients required to allow the microorganism of the invention to grow under the appropriate conditions (e.g. temperature, etc.); the culture medium may be in a solid, semi-solid or liquid state. In a particular embodiment, said culture medium is a glucose-enriched medium (YPD), an ethanol-enriched medium (YPE) or any other appropriate culture medium known to persons skilled in the art. The microorganism of the invention may be cultured in any culture medium suitable for the growth of said microorganism under suitable conditions, for example at a temperature, preferably, of between 15°C and 35°C, advantageously with stirring, for example at between 10 and 300 revolutions per minute (rpm).

In order to obtain a biologically pure culture of a microorganism of the invention, in a particular embodiment said microorganism of the invention can be cultured in a culture medium suitable for yeasts, for example a glucose-enriched medium (YPD) [20 g of peptone, 10 g of yeast extract and 20 g of glucose].

Consequently, in another aspect, the invention relates to a biologically pure culture of the microorganism of the invention, hereinafter "culture of the invention". Said biologically pure culture of said microorganism of the invention may be obtained by conventional methods as discussed previously.

In another aspect, the invention relates to a culture medium comprising the microorganism, further comprising 25% (v/v) orange juice and 1% (w/v) of a carbon source, hereinafter "culture medium comprising orange juice". Orange juice is a liquid fruit juice obtained by squeezing the inside of oranges *(Citrus sinensis).* Natural orange juice is obtained by breaking the mesocarpial sacs of the fruit and extracting the juice in a process that is undertaken industrially by gently breaking the shell so that the essential oils do not mix with the juice; this product contains between 0.1% and 20% pulp by volume (v/v) depending on the machinery used for the extraction thereof. The pH of orange juice is between 3 and 4, typically around 3.5, with this parameter being highly dependent on the ecological zone from which the fruit is obtained and its degree of maturity.

The composition of the culture medium comprising orange juice may vary over a broad range; however, in a particular embodiment, the approximate composition of said culture medium comprising orange juice (initial) preferably comprises:

| | |
|---|---|
| Soluble solids and organic acids | 11 ± 1 °Bx |
| pH | 3.5 ± 0.5 |
| % pulp | Between 0.1 and 20% v/v |
| Total Sugars | 40 - 120 g/L |
| Reducing | 30 - 60 g/L |
| Glucose | 15 - 30 g/L |
| Fructose | 15 - 30 g/L |
| Non-reducing | 30 - 60 g/L |
| Total acidity | 0.8 ± 0.2 g anhydrous citric acid/100 mL |

A person skilled in the art would understand that the specific composition of the orange juice will vary, amongst other factors, on the geographical location of the orange crop, the time of year and variety of orange, etc. as all these factors are dependent on the degree of maturity of the fruit. The variety of citrus fruit that can be used to produce the orange juice used as culture medium includes, but is not limited to, the varieties navel orange, navelate, salustiana, grapefruit, bitter orange, etc.

The orange juice to be used in the culture medium comprising orange juice for culture of a microorganism of the invention may have various origins. Said orange juice to be used in the culture medium comprising orange juice may be obtained from, but not limited to, fresh orange juice, pasteurised orange juice, frozen orange juice, concentrated orange juice, etc. If said orange juice is obtained from a concentrated orange juice, said concentrated orange juice may be diluted by addition of water to achieve the desired values of the various parameters considered herein and, if necessary, may be rectified to comply with the desired fermentation parameters indicated in the table above, mainly as regards reducing sugar concentration. In a particular embodiment, the orange juice is microbiologically stable; to this end, said culture medium comprising orange juice may be subjected to a heating process to reduce the pathogens that may be contained therein, for example a pasteurisation process. In a further particular embodiment the culture medium comprising orange juice may be rectified, if necessary, by adding sugars and/or modifying the pH.

In a particular embodiment, said culture medium comprising orange juice is wholly or partially composed of orange juice. In a further particular embodiment, said culture medium comprising orange juice comprises orange juice together with water. In this latter case, the proportion of water to orange juice, by volume, may vary over a wide range; said water:orange juice proportion may be, but is not limited to, 1:1, 1:2, 1:3, 1:4, 1:5, 1:6, 1:7, 1:8, 1:9, 1:10 (v:v) and even higher. Alternatively, the water may be present in a higher proportion than the orange juice, for example a water:orange juice proportion of 2:1, 3:1, 4:1, 5:1, 6:1, 7:1, 8:1, 9:1, 10:1 (v:v) and even higher. In the event that the culture medium comprising orange juice is not a pure or substantially pure orange juice, addition of reducing sugars, such as glucose and/or fructose, may be necessary to ensure that the carbon source is not limiting. In that case, the amount of reducing sugars to be added may vary over a wide range; in general, one or more of said reducing sugars may be added to a final concentration of between 0.2% and 5% (w/v); in a particular embodiment, glucose and/or fructose is added to a final concentration of approximately 1% (w/v).

In a further aspect, the invention relates to the use of said microorganism of the invention or said culture of the invention to perform alcoholic fermentation. As used herein, the term "alcoholic fermentation" (also known as ethanol fermentation or even ethanolic fermentation) generally refers to a biological fermentation process in the absence of air caused by the activity of one or more microorganisms (specifically, in this case, the microorganism of the invention) which process carbohydrates (e.g., glucose and/or fructose) to obtain ethanol, CO₂ and ATP as final products which, in general, are consumed by the microorganisms themselves as part of the anaerobic cellular energy metabolism thereof. In a particular embodiment, the microorganism of the invention, or a culture of the invention, is used to process one or more reducing sugars, for example glucose and/or fructose, in the absence of air.

### Growth method for the microorganism of the invention

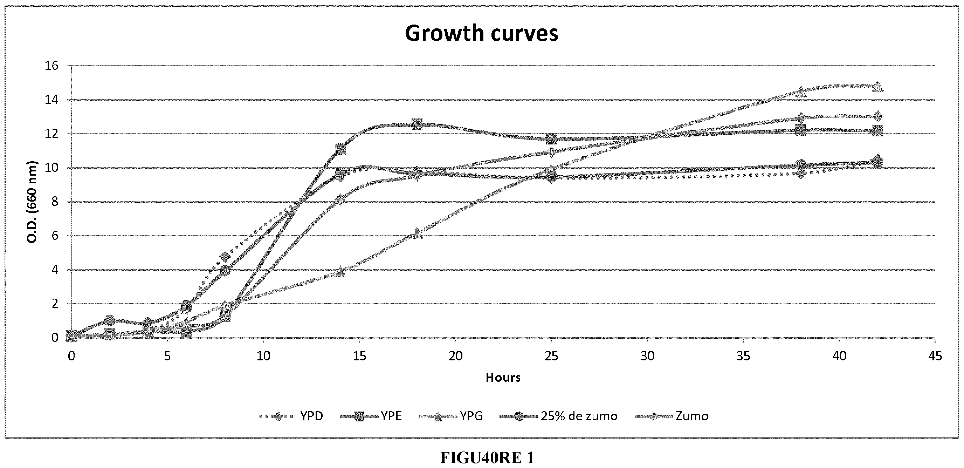

The inventors have highlighted that the microorganism of the invention is able to grow in a culture medium comprising orange juice in a similar manner as in an enriched medium, for example YPD, as shown in example 2 and figure 1.

Consequently, another aspect of the invention relates to a growth method for a microorganism of the invention, or a culture of the invention, hereinafter "first method of the invention", comprising inoculation of said microorganism or culture in a culture medium comprising orange juice.

A person skilled in the art fully understands the inoculation techniques for a yeast (microorganism of the invention) in a culture medium.

Once the microorganism of the invention has been inoculated in a culture medium comprising orange juice, the first method of the invention also includes the culture thereof to promote growth of the microorganism of the invention. The conditions required for culturing the microorganism of the invention *(Pichia kluyveri*) are fully understood and/or may readily be determined by a person skilled in the art. Advantageously, the microorganism of the invention is cultured at a temperature of between 15°C and 35°C, preferably at approximately 30°C. Moreover, advantageously, the microorganism of the invention is cultured while stirring; although said stirring may vary over a wide range, in a particular embodiment, said microorganism of the invention is cultured at a stirring rate of between 10 and 300 rpm, preferably between 100 and 250 rpm, and typically at approximately 220 rpm.

In a particular embodiment, the first method of the invention comprises a prior stage involving initial inoculation of the microorganism of the invention in an appropriate culture medium, on a small scale and in a small volume (pre-inoculum) and, once sufficient biomass has been achieved, transferring said microorganism of the invention into a culture medium comprising orange juice on a larger scale. To this end, in general, the process starts with a pure pre-inoculum of the microorganism of the invention, which is cultured under suitable conditions, for example at a temperature of between 15°C and 35°C, preferably at approximately 30°C, while stirring at a rate of between 10 and 300 rpm, preferably at between 100 and 250 rpm, advantageously at around 220 pm. Although practically any suitable culture medium for growth of the microorganism of the invention can be used as a culture medium for the pre-inoculum, in a particular embodiment said culture medium used for the pre-inoculum is YPD (2% peptone, 2% glucose and 1% yeast extract) or YPG (2% peptone, 2% glycerol and 1% yeast extract) Alternatively, in another particular embodiment, said pre-inoculum can be inoculated and cultured directly in a culture medium comprising orange juice such that the adaptation phase to the final culture medium is reduced. Moreover, in a further particular embodiment, said pre-inoculum can be inoculated and cultured in a culture medium comprising orange juice diluted to 25% with water. In a particular and preferred embodiment, the pre-inoculum is cultured in a culture medium comprising approximately 25% orange juice (v/v), approximately 75% water (v/v) and approximately 1% (w/v) of a carbon source (glucose and/or fructose).

Subsequently, after having achieved sufficient biomass in the pre-inoculum and that said pre-inoculum is in the exponential growth phase, it is transferred to the final culture medium comprising orange juice for culture on a larger scale. The pre-inoculum may be added directly to the final culture medium comprising orange juice or, alternatively, on occasions, said pre-inoculum may need to be subjected to centrifugation beforehand, as is the case, for example, when the pre-inoculum has been grown in a culture medium other than the culture medium comprising orange juice, such as YPD, YPE or YPG; in this case, it is advisable to subject the product to centrifugation and several washes in order to eliminate the majority of said culture medium (YPD, YPE or YPG).

In a particular embodiment, the culture inoculation load is between 0.05 and 0.2 absorbance units per millilitre (AU/mL) as measured by spectrophotometry at a wavelength of 660 nm. In a preferred embodiment, said absorbance value is 0.2 AU/mL, which corresponds to approximately 1x10⁵ cells/mL. Said ratio is effective if the pre-inoculum is in the exponential growth phase.

The specific culture conditions for a microorganism of the invention (P. *kluyveri*) in a culture medium comprising orange juice are known to, or may readily be determined by, a person skilled in the art. Preferably, the microorganism of the invention is cultured at a temperature of between 15°C and 35°C, preferably at approximately 30°C, while stirring at a rate of between 10 and 300 rpm, preferably at between 100 and 250 rpm, advantageously at around 220 pm.

Methods for determining whether the microorganism of the invention is growing suitably in the culture medium comprising orange juice include those methods in which the cell biomass is determined prior to growth and after an appropriate growth time and comparing said cell biomass before and after. To determine the biomass, and consequently whether the growth method has worked, the person skilled in the art known various methods belonging to the prior art, such as determination of the optical density of the culture in which the microorganism of the invention is growing at 660 nm.

### Method for obtaining an orange juice by-product

In a further aspect, the invention relates to a method for obtaining an orange juice by-product, hereinafter "second method of the invention", comprising inoculation of a microorganism of the invention, or a culture of the invention, into a culture medium comprising orange juice under conditions that allow fermentation of the reducing sugars in said orange juice. The implementation of said second method of the invention comprises the performance of various steps, which are described below.

In a first step [step (i)], the second method of the invention comprises the inoculation of a microorganism of the invention, or a culture of the invention, into a culture medium comprising orange juice. Said step (i) is common to the first method of the invention, therefore the features thereof regarding the microorganism (or culture), culture medium comprising orange juice and inoculation are included here for reference to the extent that they are applicable to the second method of the invention. Briefly, in a particular embodiment, a pre-inoculum of a microorganism of the invention is initially grown in a suitable culture medium at a temperature of between 15°C and 35°C, preferably at approximately 30°C, optionally with stirring, in a small volume to obtain sufficient biomass, and subsequently transferred to a larger volume of culture medium. In a particular embodiment, said culture medium is a culture medium comprising orange juice. In a further particular embodiment, said pre-inoculum is cultured in a culture medium comprising approximately 25% orange juice (v/v), approximately 75% water (v/v) and approximately 1% (w/v) of a carbon source (glucose and/or fructose).

Once sufficient biomass has been obtained from growth of the pre-inoculum, the pre-inoculum culture is transferred into a culture medium comprising orange juice, in a recipient suitable for performing alcoholic fermentation [step (ii)]. The pre-inoculum may be added directly to the final culture medium comprising orange juice or, occasionally, may need to be subjected to a prior treatment, for example centrifugation. In general, if the pre-inoculum has been grown in a culture medium other than a culture medium comprising orange juice, such as YPD, YPE or YPG, it is advisable to subject the pre-inoculum to a treatment normally comprising a centrifugation and various washes in order to eliminate the majority of said medium. In a particular embodiment, the culture inoculation load is between 0.05 and 0.2 AU/mL, as measured by spectrophotometry at a wavelength of 660 nm. In a preferred embodiment, said absorbance value is 0.1 AU/mL, which corresponds to approximately 1x10⁵ cells/mL. Said ratio is effective if the pre-inoculum is in the exponential growth phase.

The recipient comprising the culture medium comprising orange juice into which the pre-inoculum culture has been transferred is a recipient suitable for performing alcoholic fermentation. In a particular embodiment, said recipient in which alcoholic fermentation is performed is a tank, bottle or flask and the culture is maintained under anaerobic conditions or, alternatively, with air, preferably sterile, in the neck. In a particular embodiment, said recipient is configured to allow the gas produced to escape but to prevent air from entering, whereas in another particular embodiment, said recipient is configured to allow sterile air to enter. In a preferred embodiment, the culture is stirred either occasionally or continually or recirculation of the lower part of the fermentation broth (culture) to the upper part is allowed, or a current of sterile air is incorporated in order to homogenise the culture, thus allowing, amongst others, the bioavailability of the nutrients to be increased. Due to the presence of pulp, the microorganisms of the invention are dragged to the bottom of the recipient during sedimentation, thus resulting in a concentration gradient for said microorganisms, with the highest concentration thereof being found in the lower part of the recipient (e.g. fermenter). Consequently, homogenisation of the fermentation broth (culture) by conventional methods, such as methods that restrict sedimentation by, for example, techniques that provide movement to the fermentation broth or culture (e.g. stirring, recirculation, incorporation of a current of sterile air, etc.), allows the microorganism of the invention to ferment in those regions of the recipient (e.g. fermenter) in which the greatest quantity of pulp is found, with the fermentation of juices containing greater quantities of pulp resulting in more bitterness, in other words, in general, higher quantities of pulp lead to more bitterness after fermentation. In a particular embodiment, said movement can be achieved by stirring, for example continuous stirring (e.g. involving the use of blades inside the fermenter, orbital stirring, etc.) or by recirculation of the lower fraction in the fermenter onto the upper part, or by incorporation of sterile air. etc. In a particular and preferred embodiment, stirring during the alcoholic fermentation process is performed continually within in a range that may vary widely, for example, but not limited to, between 60 and 150 rpm, preferably 100 rpm, to ensure correct homogenisation of the contents. Stirring throughout alcoholic fermentation provides, amongst other advantages, homogenisation of the physicochemical and microbiological content of the fermenter. Advantageously, stirring should be performed with the minimum possible aeration of the fermentation broth to minimise the effects of oxygen on the antioxidants or metabolism of the yeasts. Contact between the antioxidants contained in the broth and oxygen reduces the nutritional character thereof. The effects of oxygen in contact with the yeast imply a metabolic change from fermentation (metabolism of interest) to respiratory metabolism (not of interest in this procedure).

In a particular embodiment, the microorganism of the invention may be combined with other yeasts that also participate in, or help, fermentation; said yeasts may be inoculated sequentially or at the same time as the microorganism of the invention. Said yeasts may be yeasts known in the prior art that are able to ferment any sugar, including both reducing and non-reducing sugars, and are able to generate a final product with low alcohol content and good organoleptic properties.

The duration of the alcoholic fermentation may be determined by a person skilled in the art and is directly proportional to the concentration of sugars that can be fermented by the microorganism of the invention present in the medium and inversely proportional to temperature. In a particular embodiment, the appropriate temperature for performing the alcoholic fermentation using a microorganism of the invention is in the range 15°C to 35°C, preferably between 20°C and 30°C. Under these conditions, alcoholic fermentation can be maintained for a period of time that may vary over a broad range, generally several hours or days; for example, under the conditions indicated, alcoholic fermentation can be maintained for a period of, but not limited to, between 1 hour and 20 days, typically between 12 hours and 10 days, normally between 1 day and 5 days, for example for 1, 2, 3, 4 or 5 days. Once the reducing sugars present in the culture medium have been wholly or partially depleted and ethanol and, optionally, other flavouring compounds have been produced, alcoholic fermentation can be considered to be complete. The ethanol concentration in the final fermented product may vary over a broad range depending, amongst other factors, on the concentration of reducing sugars present in the starting orange juice present in the culture medium; however, in a particular embodiment, the concentration of ethanol present in the final fermented product is between 0.1% and 3.4% (v/v). As partial fermentation of the sugars is usually obtained, there is generally no need to add sucrose or other sweeteners to the final product.

Once the alcoholic fermentation process has finished, the product resulting from step (ii) [fermented product] can, optionally be subjected to a treatment to remove or eliminate, either wholly or partially, the solids in suspension that may be present in said product [step (iii)]. Examples of solids in suspension present in the product obtained from step (ii) include, but are not limited to, microorganisms of the invention (and optionally coadjuvant yeasts that may have been added), pulp residues [step (iii)]. The removal or elimination, either whole or partial, of said solids in suspension may be performed by any conventional technique known to a person skilled in the art. In a particular embodiment, said removal or elimination, either whole or partial, of said solids in suspension may be performed by applying the following treatments, either alone or in combination:
(a) Centrifugation, in order to provoke sedimentation of the solids or particles of highest density and retain the supernatant; in a particular embodiment, said centrifugation is an industrial centrifugation, the conditions of which may be chosen by a person skilled in the art to optimise the result and may vary in a broad range, for example between 1,000 g and 10,000 g, typically approximately 5,000 g, for a period of between 1 and 30 minutes; in a further particular embodiment, said centrifugation is performed at 3,000 g for approximately 5 minutes.
(b) Decantation and disposal of the precipitate; decantation is a physical method for separating heterogeneous mixtures in which the densest particles are separated from the least dense. If this method is performed, a subsequent maturation is not required. Advantageously, decantation is performed cold, typically at a temperature of between 1°C and 8°C, preferably at approximately 4°C, for a varying length of time, normally of the order of several hours or days, for example between 1 hour and 15 days; however, in a particular embodiment, said decantation is performed at approximately 4°C over a period of between 12 hours and 10 days.
(c) Clarification using a substance with clarifying properties, such as bentonite, gelatine or any other suitable compound, such as a suitable flocculant, and disposal of the precipitate. This method may be considered to be a modification of the decantation method in which flocculants that can bind to solids, thereby increasing the density thereof and increasing sedimentation, are added.
(d) Cross-flow filtration with 0.2-0.45 µm filters, with an aspiration pressure of 2 bar (2x10⁵ Pa) and an outlet pressure of 0.5 bar (0.5x10⁵ Pa). This process allows the effluent to be recovered. Cross-flow filtration is characterised by fast circulation of the liquid tangentially to a membrane or filter. In this manner, the membrane is cleaned during filtration, thus allowing it to function continuously with stable operational features (composition, flow, etc.). The permeate retained can be pasteurised, thus eliminating the microorganisms and partially degrading the pulp and resulting in a more attractive product for consumption as the liquid and solid phases are less differentiated. Finally, the effluent is mixed with the permeate to give a sterile product containing pectins that stabilise the foam as well as antioxidants and a pleasant smell, taste and colour. The presence of pectins in the pulp means that less CO₂ is required for carbonation as said pectins improve the stability.

If the above-mentioned treatments (a), (b) and/or (c) are used to wholly or partially remove or eliminate solids in suspension, it is advantageous to submit the resulting product to a subsequent pasteurisation treatment to eliminate the microbiological load. However, treatment (d), cross-flow filtration with 0.2-0.45 µm filters, also eliminates microorganisms, therefore the final product would already be sterilised and a subsequent pasteurisation treatment would not be necessary. In a preferred embodiment, decantation treatment (b) is applied followed by pasteurisation. Pasteurisation is a heating process applied to products comprising a liquid phase in order to reduce the pathogens that may be contained therein. The pasteurisation conditions may vary within a certain interval; thus, in a particular embodiment, said pasteurisation is undertaken by heating rapidly to a temperature of between 70°C and 80°C for a time of between 15 and 120 seconds, followed by flash cooling; as an illustrative but non-limiting example, in a specific embodiment, said pasteurisation is undertaken by heating rapidly to a temperature of between 78°C and 80°C for approximately 30 seconds, followed by flash cooling or, alternatively, by heating rapidly to a temperature of between 70°C and 75°C for a time of between 15 and 30 seconds, followed by flash cooling.

Once the solids have been wholly or partially removed or eliminated, the product obtained in step (iii) can be subjected to a maturation treatment [step (iv)] in which the fermented product is stored cold, in other words at a temperature of between 1°C and 8°C, typically between 2°C and 6°C, for example at approximately 4°C, for a period of between 1 and 10 days in order to eliminate certain volatile compounds and decantable solids at said temperature. The aim of this treatment is to eliminate those solids that would form precipitates at the temperature at which the drink will be ingested. If so wished, one or more compounds that provide different properties and/or features may be added, as described below.

If so wished, the matured fermentate or fermented product obtained after the maturation treatment [step (iv)] may be subjected to an additivation and homogenisation process [step (v)] to achieve the desired physical stability (suspension of particles that tend to precipitate) and prevent, reduce or minimise the colour changes resulting from oxidation of the final product during the shelf-life thereof without causing important modifications to the organoleptic features (e.g. taste, viscosity, etc.).

The physical stability of the product obtained in step (iv) may be achieved by addition of a physical stabiliser suitable for food use that provides physical stability to the final product by limiting the sedimentation of any solids that may be present in said final product; examples of said physical stabilisers suitable for food use include, nut are not limited to, gums, for example, gellan gum (E-418), pectins (E-440), etc. In general, said additives are intended to ensure that sediment does not form in the final product due to precipitation of the solids contained therein (e.g. pulp residues, etc.). The concentration of physical stabiliser in the product obtained from step (iv) or in the final product, if added, may vary in a broad range, generally of between 0.01% and 1% (w/v), typically around 0.4% (w/v).

Moreover, as mentioned previously, the darkening or browning of the product obtained in step (iv), or the optionally physically stabilised product, or the final product can be prevented, reduced or minimised, if so wished, by addition of a suitable antioxidant, such as an antioxidant for food use; almost any antioxidant for food use that allows said darkening or browning to be prevented, reduced or minimised may be used during application of this invention; however, in a particular embodiment, said antioxidant is selected from amongst ascorbic acid, citric acid and salts thereof, for example, sodium citrate, calcium citrate, etc., tartaric acid, etc. and mixtures thereof. In a specific embodiment, said antioxidant is ascorbic acid as said compound is found naturally in orange juice. If at least one antioxidant for food use is added to prevent, reduce or minimise darkening or browning of the product obtained in step (iv), or the optionally physically stabilised product, or the final product, the concentration of antioxidant in said product(s) may vary over a broad range, generally between 0.01% and 2% (w/v), typically around 0.1% (w/v).

Optionally, if so wished, the additioned product may be subjected to a stirring process to promote subsequent homogenisation. In a particular embodiment, said homogenisation is performed at between 100 bar (100 x 10⁵ Pa) and 500 bar (250 x 10⁵ Pa), preferably at approximately 250 bar (250 x 10⁵ Pa) to ensure correct dispersal and hydration of the additives incorporated.

The additivated and homogenised product obtained in step (v) is subjected to a moderately mild heat treatment [step (vi)], typically at a temperature of 100°C or less, for s short period of time, typically of the order of a few seconds, for example between 5 and 120 seconds, normally between 10 and 45 seconds, preferably between 15 and 30 seconds, to reduce the microbial population present therein and, to this end, to achieve microbiological stability in the final product in order to be able to store said product at room temperature. In a particular embodiment, the additivated and homogenised product obtained in step (v) is subjected to a heat treatment (e.g. pasteurisation) with a maintenance temperature of between 70°C and 80°C for a time period of between 5 and 120 seconds, and subsequently cooled rapidly to room temperature, advantageously to a temperature of between 78°C and 80°C for approximately 30 seconds, followed by fast cooling to approximately 20°C. The average calculated lethality value after this treatment is 16.

Finally, if so wished, and either before or after the heat treatment of step (vi), the additivated and homogenised product obtained in step (v) may be subjected to a gasification or carbonation treatment [step (vii)], which comprises the addition of carbon dioxide to achieve a pressure of between 0.1 and 2.5 bar (0.1-2.5 x 10⁵ Pa), preferably 0.44 bar (0.44 x 10⁵ Pa), at equilibrium. In a particular embodiment, this treatment is performed at a temperature of between 1°C and 20°C, preferably between 2°C and 8°C, and normally at approximately 4°C, in a specific recipient for gasification. This pressure results in a preferred ratio of 2 volumes of CO₂ per unit volume of drink. Gas exchange between the phases (liquid-gas) is allowed to reach equilibrium and, if necessary, further CO₂ is added to reach a pressure of around 0.44 bar (0.44 x 10⁵ Pa). The carbonation time will depend on the type of recipient in which said process is performed as the liquid/gas exchange area has an effect and will be sufficient to reach the desired equilibrium pressure. In a preferred embodiment, for more efficient carbonation, carbonation is allowed to proceed for 24 hours at 4°C with the largest possible exchange area. Alternatively, carbonation may be performed by adding dry ice (solid carbon dioxide), preferably at a concentration of 1 to 20, preferably of 2 to 10, more preferably of approximately 6 g of dry ice/litre of product.

In the event that the final product (which may or may not be carbonated) is sterilised, said product may be stored at room temperature for an extended time period, of the order of several months, without contamination problems. Commercialisation of the product with no need for a cold chain reduces the operating costs, thereby increasing the possibility of better sales and distribution.

The second method of the invention leads to a different orange juice by-product to those described in the prior art and with appropriate and very good organoleptic properties for consumption thereof. Thus, in a further aspect, the invention relates to an orange juice by-product comprising the microorganism of the invention presenting an ethanol concentration of between 0.1% and 3.4% (v/v) produced via the second method of the invention. The final ethanol concentration in said orange juice by-product provided by the invention, comprising the microorganism of the invention, is preferably 2.5%. In a particular embodiment, orange juice with a pulp concentration of 7% to 15% (v/v) is used as the features of the final product are appropriate for the consumption thereof as it is not too bitter.

The term "orange juice by-product" as used herein relates to a product generated by alcoholic fermentation of orange juice by a microorganism of the invention, comprised in said by-product, which presents an ethanol concentration of between 0.1% and 3.4% (v/v). In a particular embodiment, said product comprises non-reducing sugars, such as sucrose, at a concentration of approximately 50 g/L, thus meaning that sweeteners need not be added.

Moreover, additional products such as flavouring compounds, acidifiers, dyes, preservative, antioxidants, emulsifiers, thickeners, pulp stabilisers, additives to improve the properties of the product or any other product of interest for industry that increases the value of said product, or any combination thereof, may be added. Suitable compounds for addition to the final product include, but are not limited to, taurine, caffeine, theophylline, pectin, gums, carboxymethyl cellulose (CMC), carrageenans, starch or derivatives thereof, cellulose, alginic acid, sodium alginate, potassium alginate, ammonium alginate, calcium alginate, propylene glycol alginate, sorbic acid, sodium sorbate, potassium sorbate, calcium sorbate, benzoic acid or benzoates, sulfites, ascorbic acid or ascorbates, tocopherols, lactic acid or lactates, citric acid or citrates, tartaric acid or tartrates and phosphoric acid or phosphates or polyphosphates.

In a particular embodiment, the features of the final product are as follows:

| | |
|---|---|
| **Soluble solids and organic acids** | Initial up to 7 °Bx |
| **pH** | 3.5 ± 0.5 |
| **% pulp** | Depends on post-fermentation treatment |
| **Total Sugars** | 30 - 90 g/L |
| **Reducing** | Up to 30 g/L |
| **Glucose** | Up to 15 g/L |
| **Fructose** | Up to 15 g/L |
| **Non-reducing** | 30 - 60 g/L |
| **Total acidity** | 0.8 ± 0.2 g anhydrous citric acid/100 mL |
| **Ethanol** | 0.1-3.4% v/v |

The main volatile compounds in the orange juice by-product are preferably as follows:

| **Compound** | **Mean concentration (mg/L)** |
|---|---|
| Acetaldehyde | 5.65 |
| Methyl acetate | 0.54 |
| Ethyl acetate | 501.95 |
| Methanol | 37.65 |
| 1-propanol | 27.23 |
| Isobutanol | 39.07 |
| Isoamyl acetate | 9.04 |
| 2-Methyl-1-butanol | 11.15 |
| 3-Methyl-1-butanol | 30.75 |

Some illustrative examples that highlight the features and advantages of the invention are described below. However, said examples must not be considered to limit the subject matter of the invention.

### EXAMPLE 1

### Isolation and characterisation of Pichia kluyveri (CECT 13055)

### I. MATERIALS AND METHODS

### Fruit

Samples were taken from the following citrus fruit varieties in the search for yeasts from the citric environment: navel, navelate, salustiana, grapefruit and bitter orange.

All fruits were taken from the Vega del Guadalquivir (Seville), the setting with the greatest quantity and variety of citrus fruits in the experimental zone. The regions from which samples were taken had been 30 days with no chemical treatment that may have limited spontaneous growth of microbiota in the environment.

Three types of samples were taken:
(1) Whole oranges,
(2) Oranges whose skin had been scored 7 days previously, and
(3) Oranges in a state of spontaneous decomposition placed on the ground.

### Culture media

Samples were cultured on plates with a medium that allowed the growth of any yeast deposited thereon. Said medium is an enriched medium that uses glucose as carbon source, the composition of which is as follows:
For a total volume of 1,000 mL: 20 g of peptone, 10 g of yeast extract, 20 g of agar and 20 g of carbon source (glucose). Said medium was subsequently sterilised in an autoclave. A total of 20 mL of the above medium was dosed onto each Petri dish under sterile conditions.

### Culture of samples on plates

Different regions of the fruit samples received in the laboratory were cultured on culture medium plates. Thus, depending on the sample type, the procedure was as follows:
- Whole oranges: Sterile toothpicks were passed over the skin of the orange and placed on the culture medium plate. The fruits were also opened and the region between the skin and the pulp (region between the epicarp and the mesocarp) rubbed with different toothpicks. Small volumes of juice were removed directly from the mesocarp using a micropipette and seeded on the plates.
- Oranges whose skin had been scored 7 days previously: Sterile toothpicks were passed over the skin of the orange and the scores made previously and placed on the culture medium plate. The fruits were also opened and the region between the skin and the pulp (region between the epicarp and the mesocarp) rubbed with different toothpicks. Small volumes of juice were removed directly from the mesocarp using a micropipette and seeded on the plates.
- Oranges in a state of spontaneous decomposition placed on the ground: Sterile toothpicks were passed over the skin of the orange and placed on the culture medium plate. Small volumes of juice were removed directly from the mesocarp using a micropipette and seeded on the plates.

### II. RESULTS

### Identification of the yeast strains

A search for those yeasts that were able to grow was performed for all plates tested (a total of 50). Other moulds and bacteria also grew on the plates as well as yeasts.

An initial visual screening of the plates was performed in search of yeast colonies (whiter and less shiny than bacterial colonies). Once those colonies that could visually be identified as yeasts had been identified, or about which there were doubts, the colonies were viewed under a microscope to verify that they were actually microorganisms of interest. Yeasts are larger than bacteria, have a more rounded morphology and are able to replicate by gemmation.

The yeast strains identified were seeded on new plates for purification and maintenance for subsequent testing.

### Selection of yeasts of interest

Orange juice fermentations were tested, on a small scale (250 mL), for different times, using the entire battery of yeasts obtained in the isolation and identification process and discarded if they presented an unfavourable organoleptic profile for a total of 8 tasters.

A total of 9 microorganism colonies obtained from the yeast strain isolation and identification process were used for fermentation and tasting.

After taste screening, two yeasts (strains 4N179 and 4N187) were found to provide a very good organoleptic profile. Said strains were sent to the Spanish Type Culture Collection (CECT) for molecular identification. The report indicated that both yeasts belonged to the same species *(Pichia kluyveri*)*.*

Parallel fermentations and under different conditions were performed with said two yeast strains and *Pichia kluyveri* strain 4N187 (CECT 13055) was found to provide a more constant and homogeneous product. As such, said strain was selected as the best yeast for orange juice fermentation and subsequent studies.

To determine the physicochemical properties of the yeast, growth thereof was studied in various media as it is important to understand the biochemical needs for yeast growth in order to start from a raw material that is ideal for yeast metabolism. Some of these studies included growth in different media containing different nitrogen and carbon sources, enriched media, media containing components to inhibit the growth of other microorganisms (potassium metabisulfite), etc.

### Taxonomic analysis of Pichia kluyveri

The main analyses performed routinely to characterise yeasts are related to the use of hydrocarbonated compounds, namely sugar fermentation, sugar assimilation and alcohol assimilation.

The results of these analyses for *Pichia kluyveri* were as follows:

| **Assimilation** | | | |
|---|---|---|---|
| Glucose | + | N-acetyl-D-glucosamine | N |
| Galactose | - | Methanol | - |
| L-sorbose | - | Ethanol | + |
| Sucrose | - | Glycerol | + |
| Maltose | - | Erythritol | - |
| Cellobiose | - | Ribitol | - |
| Trehalose | - | Galactitol | - |
| Lactose | - | D-mannitol | - |
| Melibiose | - | D-glucitol | - |
| Raffinose | - | α-methyl-D-glucoside | - |
| Melezitose | - | Salicin | - |
| Insulin | - | D-gluconate | - |
| Soluble starch | - | DL-lactate | +/w |
| D-xylose | v | Succinate | +/w |
| L-arabinose | - | Citrate | w/- |
| D-arabinose | - | Inositol | - |
| D-ribose | - | Hexadecane | - |
| L-rhamnose | - | Nitrate | - |
| D-glucosamⁱne | n | Free vitamin | - |

| | | | |
|---|---|---|---|
| **(+), positive;(-), negative; w, weak; v, variable (+/-, w/-); n, no data** | | | |

The results of other assimilation analyses and the determination of other features, such as growth capacity at different temperatures or Co-Q type, are listed below:

| **FERMENTATION** | *P. kluyveri var. kluyveri* |
|---|---|
| Glucose | + |
| Galactose | - |
| Sucrose | - |
| Maltose | - |
| Lactose | - |
| Raffinose | - |
| Trehalose | - |
| D-glucitol | - |
| D-glucitol | - |

| **ASSIMILATION REACTIONS AND OTHER FEATURES** | *P. kluyveri var. kluyveri* |
|---|---|
| Glucose | + |
| Galactose | - |
| L-sorbose | - |
| Sucrose | - |
| Maltose | - |
| Cellobiose | - |
| Trehalose | - |
| Lactose | - |
| Melibiose | - |
| Raffinose | - |
| Melezitose | - |
| Inulin | - |
| L-arabinose | - |
| D-arabinose | - |
| D-ribose | - |
| L-rhamnose | - |
| D-glucosamine | + |
| N-acetyl-D-glucosamine | + |
| Methanol | - |
| Ethanol | + |
| Glycerol | + |
| Erythritol | - |
| Ribitol | - |
| Galactitol | - |
| D-mannitol | - |
| D-glucitol | - |
| α-methyl-D-glucoside | - |
| Salicin | - |
| D-gluconate | - |
| Citrate | V |
| Inositol | - |
| Hexadecane | - |
| Nitrate | - |
| Nitrite | N |
| Free vitamin | - |
| 2-Keto-D-gluconate | - |
| 5-Keto-D-gluconate | - |
| Saccharic acid | - |
| Xylitol | N |
| L-arabinitol | N |
| Arbutin | N |
| 1,2-propanediol | N |
| 2,3-butanediol | N |
| Cadaverine | N |
| Creatine | N |
| L-lysine | N |
| Ethylamine | N |
| 50% glucose | N |
| 10% NaCl/5% glucose | + |
| Starch formation | - |
| Urease | N |
| Growth at 19°C | + |
| Growth at 25°C | + |
| Growth at 34°C | N |
| Growth at 37°C | V |
| Growth at 40°C | N |
| Main component of Co-Q | Co-Q 7 |

| | |
|---|---|
| (+), positive;(-), negative; w, weak; v, variable (+/-, w/-); n, no data | |

### EXAMPLE 2

### Alcoholic fermentation of orange juice by Pichia kluyveri (CECT 13055)

### I. MATERIALS AND METHODS

### Culture medium

Fermentations were performed in sterile recipients. Sterilisation was performed in an autoclave either with heat or using chemicals for said purpose. The chemicals used were 50% bleach (sodium hypochlorite) in water or 70% ethanol in water, and the recipient was rinsed prior to fermentation to remove said agents.

Commercial 250 mL bottles, 5 L aluminium barrels, polypropylene recipients of different volumes and laboratory flasks of different volumes were used as recipients, as required.

The starting orange juice contained a minimum quantity of microorganisms to rule out the proliferation of undesired organisms that could affect the fermentation process and therefore the final product. Specifically, the orange juice obtained after a pasteurisation process, and therefore free from microorganisms, was used.

The following parameters were determined for the starting orange juice: soluble solids and organic acids, pH, percentage pulp, reducing and non-reducing sugars and total acidity. Said parameters, which had to be within a certain range in order for the raw material to be considered suitable for fermentation, are described below:

| | |
|---|---|
| **Soluble solids and organic acids** | 11 ± 1 °Bx |
| **pH** | 3.5 ± 0.5 |
| **% pulp** | 7-15% v/v |
| **Total Sugars** | 40-120 g/L |
| **Reducing** | 30-60 g/L |
| **Glucose** | 15-30 g/L |
| **Fructose** | 15-30 g/L |
| **Non-reducing** | 30-60 g/L |
| **Total acidity** | 0.8 ± 0.2 g anhydrous citric acid/100 mL |

These parameters vary according to the geographical location of the crop, the time of year and citrus variety as all are dependent on the degree of maturity of the fruit. Consequently, whenever necessary, the raw material was rectified until the desired parameters described in the previous table were achieved.

The degrees Brix (°Bx) was measured by refractometry using a portable refractometer on scale of 0 to 32 °Bx. The percentage pulp was determined by adding 10 mL of juice homogenised by stirring to a graduated 15 mL tube and centrifuging at 3,000 rpm for 10 minutes. The percentage pulp was determined on the basis of the pulp precipitated and quantified by the graduations on the tube.

Reducing sugars were determined using the 3,5-dinitrosalicylic acid (DNS) colorimetric test. Non-reducing sugars were determined by acid hydrolysis of sucrose to a reducing sugar and quantification thereof as described above.

The acidity was determined by titration against 0.1 N sodium hydroxide using phenolphthalein as indicator.

Culture was performed in a reducing sugar-enriched medium prepared from orange juice containing 25% (v/v) orange juice centrifuged at 300 rpm for 5 minutes, 1% (w/v) of a carbon source (glucose or fructose) and 75% water. The culture medium contained at least 20 g/L of reducing sugars that could be assimilated by the yeast. No nitrogen source was required as the orange juice itself provides this nutrient. The culture medium was heat-sterilised in an autoclave, with a temperature plateau of 121°C, for 20 minutes to prevent contamination and obtain a pure pre-inoculum containing the strain of interest only.

### Fermentation

Starting from a pure pre-inoculum of yeast strain CECT 13055, said pre-inoculum was incubated in the culture medium described above, at 30°C, while stirring at 220 rpm.

Other culture media for growth of the starter culture, such as a medium enriched with glucose as carbon source, a medium enriched with glycerol, a medium enriched with ethanol as carbon source and undiluted orange juice, were also tested.

After testing different inoculation loads, the optimal inoculation load in the culture was found to correspond to the absorbance range 0.05 to 0.2 when measured at 660 nm. 0.1 absorbance units per millilitre corresponds to approximately 1x10⁵ viable cells per millilitre. Said ratio is effective if, at transfer of the pre-inoculum, said pre-inoculum is in the exponential growth phase. Above 0.2 the result was similar to that obtained with an absorbance of 0.1. However, below 0.05 the fermentation process was essentially non-existent and was very protracted.

The juice and yeast were introduced into the fermenter under anaerobic conditions or with air in the neck, in variable proportions, ranging from 60% to 95% volume of juice/volume of fermenter. This type of recipient allowed gas to leave but not enter, and if air entered, said air was sterile to prevent contamination with other microorganisms. A valve or other airlock-type device was used for this purpose.

Fermentation was performed under non-static conditions with movement of the matrix. Said movement was achieved in two ways: by continuous stirring (paddles inside the fermenter or orbital stirring) and by recirculation of the lower fraction in the fermenter onto the upper fraction. The stirring rate was maintained constant at 100 rpm during the fermentation process. For recirculation, sample was taken from the lower portion of the fermenter and sent to the upper portion of the fermenter using a fluid pump, where it was released. This process resulted in homogenisation of the contents. The recirculation rate varied depending on the type of fermenter as correct homogenisation of the contents was essential.

The fermentation time is directly proportional to the concentration of sugars that can be fermented by the yeast in the medium and inversely proportional to the temperature. Thus, the optimal fermentation temperature was 20°C, with a duration of 3 days. This time resulted in partial depletion of the fermentable sugars present and the production of ethanol and other flavouring compounds, thus resulting in a totally different product from the starting orange juice. The ethanol concentration in the fermentate varied with the concentration of reducing sugars present in the starting juice, although the amount of ethanol varied between 0.1% and 3.4% v/v. The enzymatic kit (alcohol dehydrogenase) manufactured by ROCHE and sold by R-Biopharm, Cat. No. 10 176 290 035 was used to determine the ethanol concentration. Ethanol can be oxidised to acetaldehyde by NAD in the presence of the enzyme alcohol dehydrogenase (ADH) to produce NADH. The NADH produced is determined spectrophotometrically at 334, 340 or 360 nm. Said determination of NADH allows the ethanol concentration to be determined indirectly. Gas chromatography was used to identify the other volatile alcohols.

A volatile compounds (aromas) profile was prepared for the final product by gas chromatography/mass spectrometry using a special injector that is able to sample the gas fraction containing said aromas.

### Post-fermentation treatment

The following operations, either alone or in combination, were tested for the post-fermentation treatment:
(1) Centrifugation at 3,000 g for 5 minutes and elimination of the precipitate.
(2) Decantation at 4°C for 1 to 10 days and elimination of the precipitate to eliminate the sedimented solids.
(3) Clarification with bentonite to increase the density of the solids in suspension and promote the sedimentation thereof, followed by elimination of the precipitate.
(d) Cross-flow filtration with 0.2-0.45 µm filters, with an aspiration pressure of 2 bar (2x10⁵ Pa) and an outlet pressure of 0.5 bar (0.5x10⁵ Pa) and retention of the effluent.
(5) Pasteurisation by heating to a temperature of between 78°C and 80°C for various times of between 10 seconds and 5 minutes.
(6) Additivation by addition of gellan gum (E-418) and/or pectins (E-440), at different concentrations of between 0.1% and 1% (w/v) to improve the physical stability of the final product by limiting phase separation and/or oxidation.

It was found that only the cross-flow filtration (4) and pasteurisation (5) treatments provided microbiological stability. The remaining post-fermentation treatments cannot be used alone, although they may be used in combination with other treatments.

As most suitable post-fermentation treatments, it was therefore concluded that decantation followed by additivation and pasteurisation were the most optimal treatments as they provided physical and microbiological stability to the final product without altering the organoleptic properties thereof.

Decantation allows the majority of solids (pulp) and yeasts to be removed. Said process is performed cold to restrict further fermentation and precipitate the majority of the solids that are decantable at this temperature.

Additivation improved the physical stability of the product.

Pasteurisation resulted in microbiological stability and halted the fermentation process, even at room temperature. This allows the product to be commercialised without needing to maintain a cold chain. The pasteurisation required to provide stability involved rapid heating, maintaining the product at a temperature of between 78°C and 80°C for 30 seconds and flash cooling.

### Carbonation

This process was performed at 4°C in a specific gasification recipient. It involved the addition of carbon dioxide to achieve a specific pressure of 0.44 bar (0.44 x 10⁵ Pa) at equilibrium. This pressure results in a ratio of 2 volumes of CO₂ per unit volume of drink. The procedure involved adding CO₂, allowing the gas exchange between the phases (liquid-gas) to reach equilibrium and, if necessary, adding further CO₂ to reach a pressure of around 0.44 bar (0.44 x 10⁵ Pa). For more efficient carbonation, carbonation was allowed to proceed for 24 hours at 4°C with the largest possible exchange area.

The carbonation procedure enhanced the organoleptic properties of the final product by providing a strongly refreshing character and making the product more attractive due to the formation of an upper layer of foam.

### II. RESULTS

The growth curves for yeast strain CECT 13055 in the exponential phase in the different culture media are shown in Figure 1.

The media in which the culture took longer to reach said exponential phase are orange juice and the medium containing glycerol as culture medium (YPG). The medium containing ethanol as carbon source (YPE) has a very pronounced exponential phase, although said phase is slightly delayed with respect to those for YPD and the medium containing 25% juice. It should be noted that the culture medium containing ethanol as carbon source is the most expensive, therefore the use thereof in the fermentation process is not recommended.

The medium containing 25% orange juice and that containing glucose as carbon source (YPD) reached the exponential phase before the others. As can be seen from Figure 1, the growth curves for these media are similar.

After comparing growth in the different media, the most optimum medium for culture of the pre-inoculum was considered to be that containing 25% orange juice slightly enriched with reducing sugars (25% (v/v) orange juice, 1% (w/v) of a carbon source (glucose or fructose) and 75% water). The reasons why said medium is considered to be the most suitable are the fact that is the cheapest, the stationary phase is achieved in a shorter time, the maximum optical density achieved is acceptable and, moreover, the time required by the microorganism to adapt to the fermentation medium is shorter and the culture does not need to be washed when changing from the pre-inoculum to the culture. As the pre-inoculum is produced in a similar medium to orange juice, there is no need for adaptation of the microorganism from growth in the pre-inoculum to growth in orange juice for subsequent fermentation.

The final product obtained after fermentation presents the following features:

| | |
|---|---|
| **Soluble solids and organic acids** | 7 °Bx |
| **pH** | 3.5 ± 0.5 |
| **% pulp** | Depends on post-fermentation treatment |
| **Total Sugars** | 30-90 g/L |
| **Reducing** | 30 g/L |
| **Glucose** | 15 g/L |
| **Fructose** | 15 g/L |
| **Non-reducing** | 30-60 g/L |
| **Total acidity** | 0.8 ± 0.2 g anhydrous citric acid/100 mL |
| **Ethanol** | 3.5% v/v |

The main volatile compounds present in the orange juice by-product are preferably as follows:

| **Compound** | **Mean concentration (mg/L)** |
|---|---|
| Acetaldehyde | 5.65 |
| Methyl acetate | 0.54 |
| Ethyl acetate | 501.95 |
| Methanol | 37.65 |
| 1-propanol | 27.23 |
| Isobutanol | 39.07 |
| Isoamyl acetate | 9.04 |
| 2-Methyl-1-butanol | 11.15 |
| 3-Methyl-1-butanol | 30.75 |

Of the compounds mentioned, those of greatest organoleptic interest for constituting fruity flavours are acetaldehyde (ripe apples), ethyl acetate (pineapple), isobutanol, isoamyl acetate (banana), methanol (fruity aroma) and 1-propanol (vinegar).

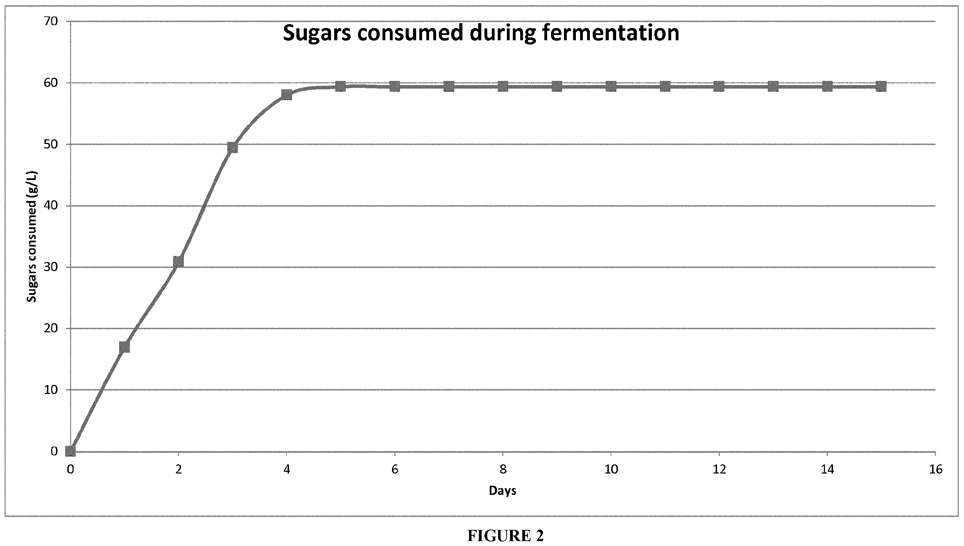

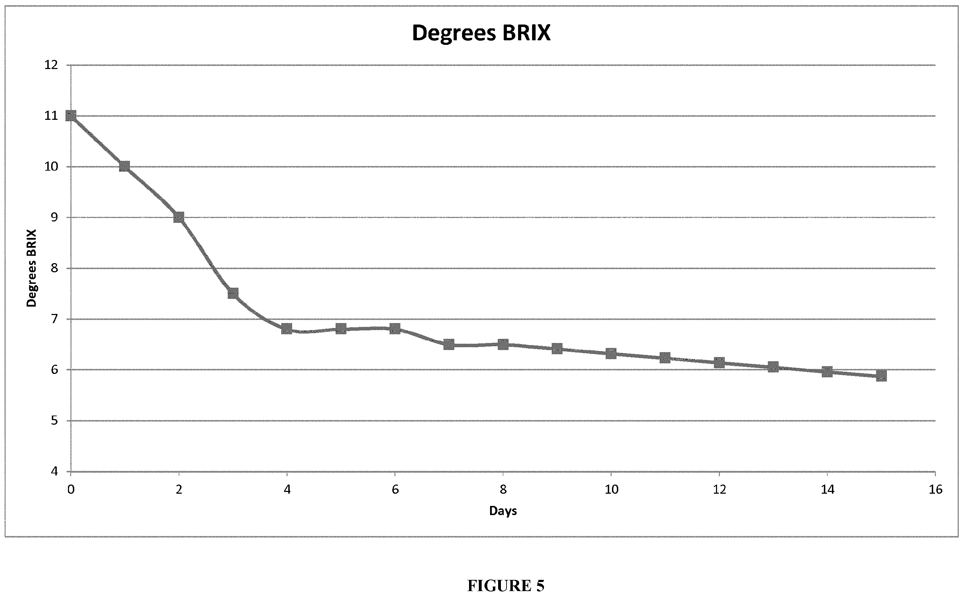

Figures 2, 3, 4 and 5 show the evolution of the ethanol concentration and degrees Brix (°Bx) during the sugar fermentation process when using the yeast strain of the invention to perform said fermentation.

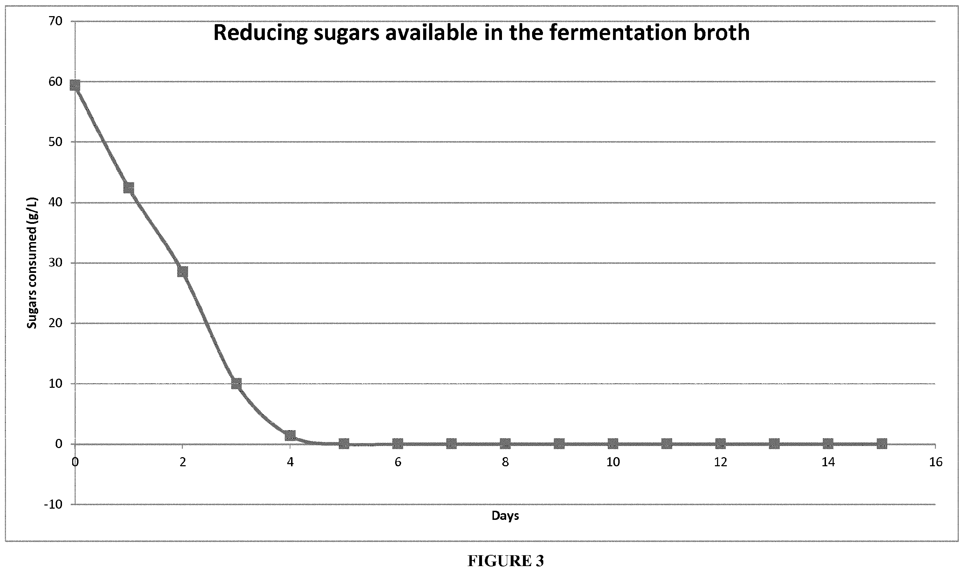

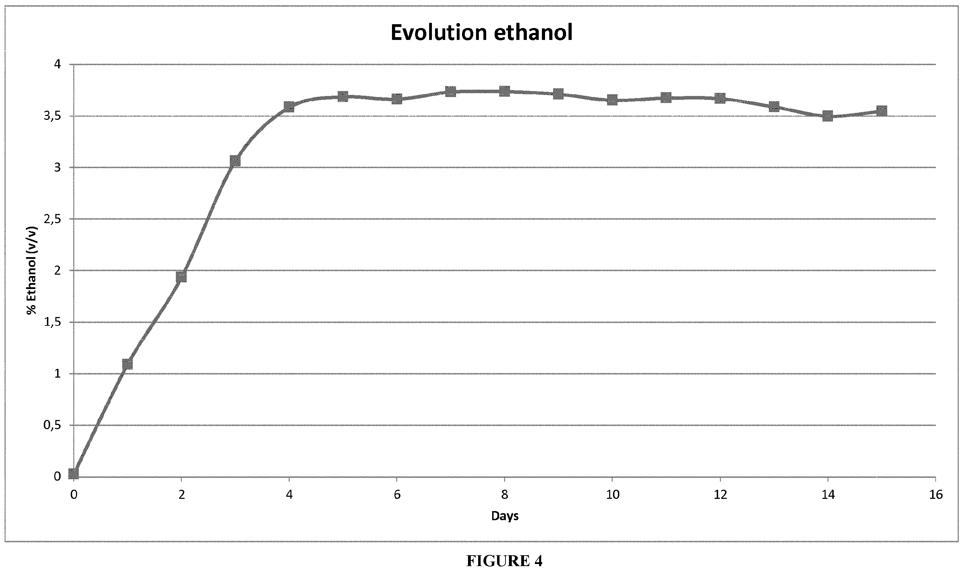

It can be seen from Figure 2 that approximately 60% of the sugars present in the culture medium have been consumed by day three of the fermentation and that said quantity does not increase further with fermentation time. Specifically, the concentration of non-reducing sugars present in the fermentation medium (sucrose) remains unchanged during the fermentation process as the yeast strain is unable to consume said sugars, with the initial and final sucrose concentrations being 48 g/L. However, as can be seen from Figure 3, the concentration of reducing sugars available has decreased dramatically by day 4 of the fermentation until said sugars are essentially depleted. As regards the ethanol concentration, it can be seen from Figure 4 that said concentration remains unchanged after day 4, in other words it remains at approximately 3.5%. Finally, the degrees Brix (°Bx), which measures the overall ratio of sucrose dissolved in a fluid decreases with fermentation time such that the starting orange juice has a value of 11 °Bx, with said value decreasing to around 7 °Bx after fermentation for four days.

Both the acidity and pH remained unchanged during the fermentation process. The acidity remained at around 0.8 g of anhydrous citric acid/100 mL and the pH at around 3.5. Although mild fluctuations were observed, they did not follow a clear trend. This is probably due to the fact that the orange juice fermented already presents an acidic pH and the yeast does not need to modify said pH in order to adapt to it.

### EXAMPLE 3

### Comparison of the fermentations performed using different yeasts

A comparative study of the stirred fermentation of orange juice with different yeast strains was performed. Specifically, the yeast strains used were as follows:
- 4N187: A *Pichia kluyveri* strain isolated from the natural setting of the citruses and deposited with CECT number 13055.
- 4N179: A *Pichia kluyveri* isolated from the natural setting of the citruses.
- *Pichia fermentans*
- *Sacchromyces cerevisiae:* a yeast traditionally used for alcoholic fermentation in winemaking, brewing and breadmaking.
- *Saccharomyces cerevisiae* var. *bayanus:* a yeast traditionally used for alcoholic fermentation in winemaking and brewing.
- *Saccharomyces carlsbergensis:* a yeast traditionally used for alcoholic fermentation in brewing.

Comparative data for these strains during stirred fermentation (at 100 rpm) are provided. All fermentations were performed at 20°C and were tested over 15 days. The parameters evaluated during fermentation were total sugars (reducing and non-reducing), ethanol and acidity.

### Reducing sugars

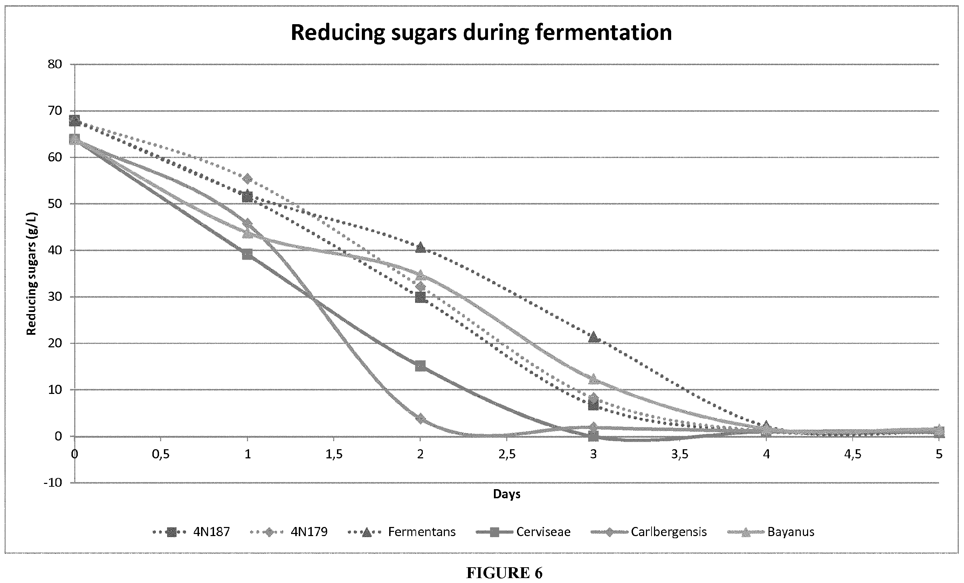

The concentration of reducing sugars, such as glucose and fructose, decreased with fermentation time for all strains. As all reducing sugars had been consumed by the end of day 5, Figure 6 shows a graph for the first 6 days of fermentation only. On day 2 of fermentation, the strains with a greater capacity to metabolise reducing sugars were *S. carlsbergensis* and *S. cereviseae,* with the other strains showing similar values. By day 3, the reducing sugar concentrations had decreased to between 0 and 10 g/L for all strains except *P. fermentans* and *S. cerevisiae* var. *bayanus.* By day 4, the concentration of reducing sugars was essentially zero for all strains.

### Acidity

The acidity of the fermentate was analysed throughout the procedure and found to remain unaltered during fermentation irrespective of whether the procedure was performed with stirring or not.

### Non-reducing sugars

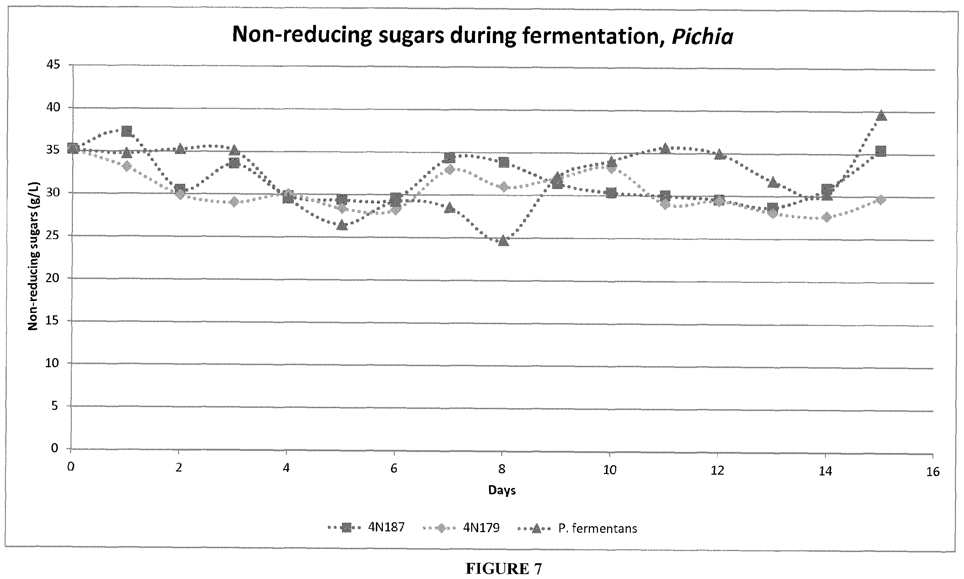

Figures 7, 8 and 9 show the evolution of a non-reducing sugar, such as sucrose, during fermentation.. Sucrose can be fermented by all strains except those of the genus *Pichia.*

As can be seen in Figure 7, the two *P. kluyveri* strains (4N187 and 4N179) present a very similar profile as regards non-reducing sugar consumption. The sucrose concentration remains essentially unchanged during the fermentation procedure with these strains, with the minor fluctuations observed probably resulting from sampling and the quantification procedure. Any effect on the metabolism of the strain would result in a clear trend during fermentation. This test confirms the inability of the *P. kluyveri* yeast strain to metabolise sucrose under stirring.

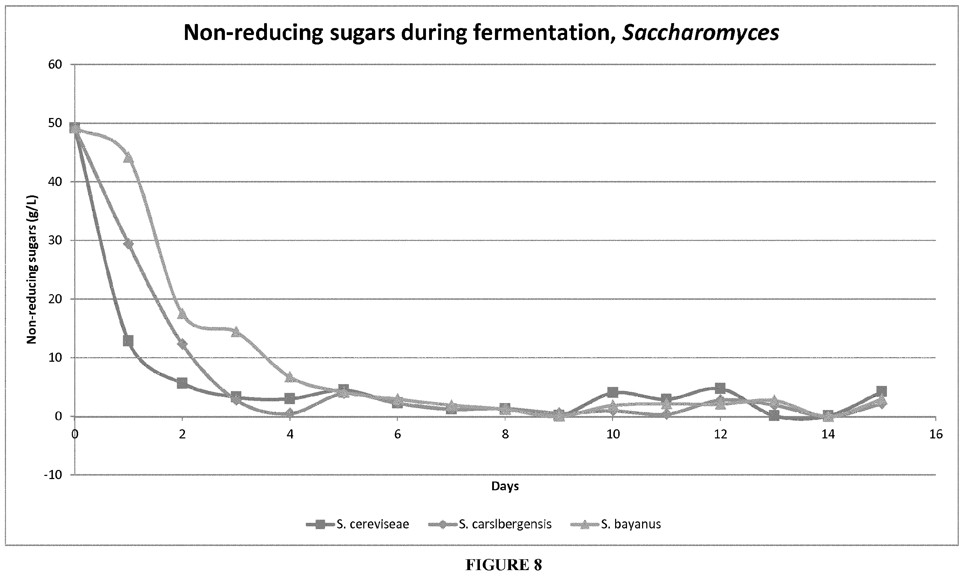

As regards the evolution of the sucrose concentration for the *Saccharomyces* strains, as can be seen from Figure 8, the sucrose concentration for these three strains on day 6 is essentially zero. In other words, as of day 6, no fermentable sugars remain in the fermentation broth as said strains are able to metabolise sucrose.

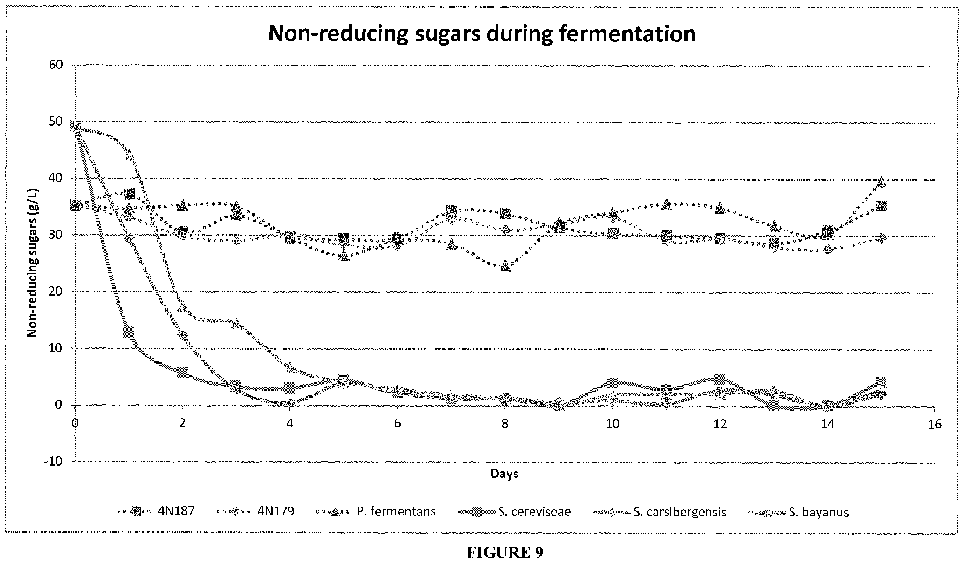

Figure 9 shows a comparison of the evolution of the sucrose concentration for all yeast strains tested. The difference between strains of the genus *Pichia* (4N187, 4N179 and *P. fermentans*)*,* which are unable to metabolise sucrose, and those of the genus *Saccharomyces (S. cereviseae, S. carlsbergensis* and *S. cerevisiae var. bayanus),* which can degrade said sugar, is evident. Within the genus *Saccharomyces, S. cerevisiae var. bayanus* presents the lowest activity and *S. cereviseae* the highest.

### Ethanol

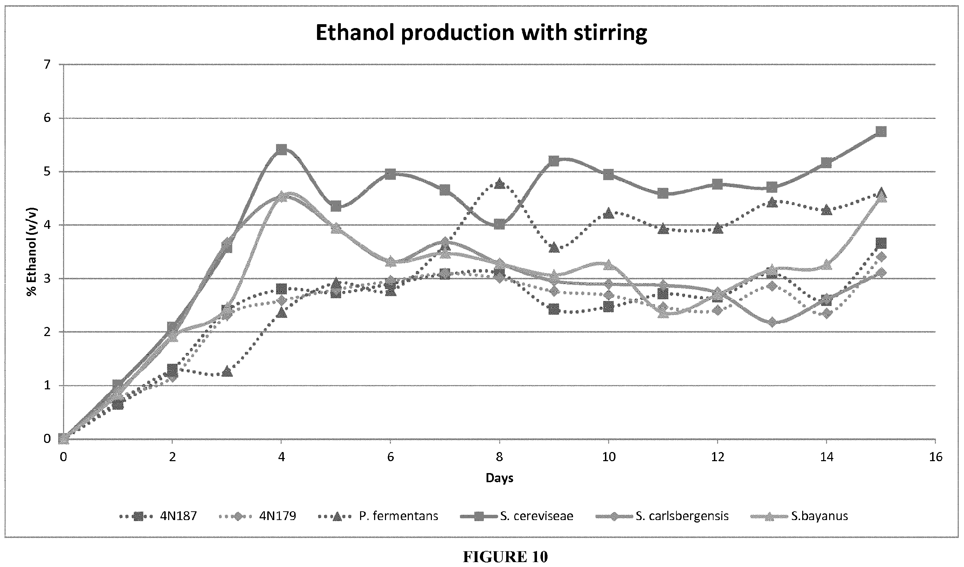

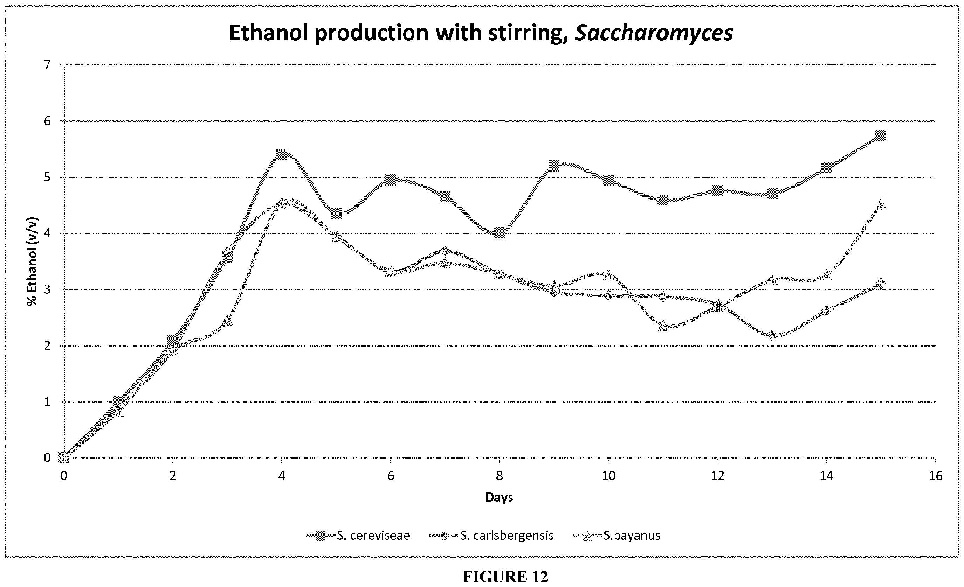

As is also the case for the consumption of non-reducing sugars (sucrose), the evolution of ethanol production follows two distinct patterns depending on the genus of yeast strain used, whether for strains of the genus *Pichia* or for strains of the genus *Saccharomyces.* Higher ethanol production levels are obtained for yeasts from the genus *Saccharomyces,* which is reasonable as said strains are able to metabolise sucrose (Figure 10 and Figure 12).

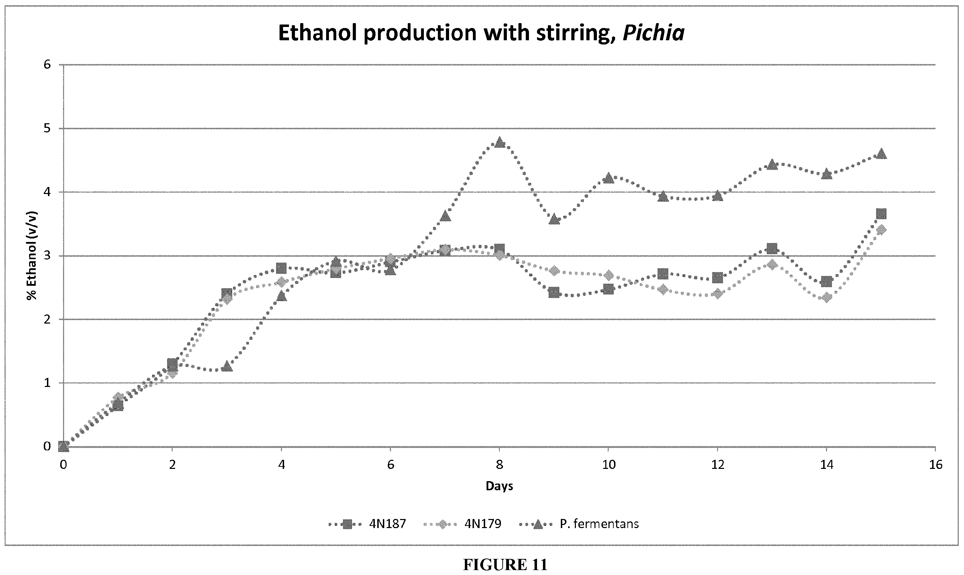

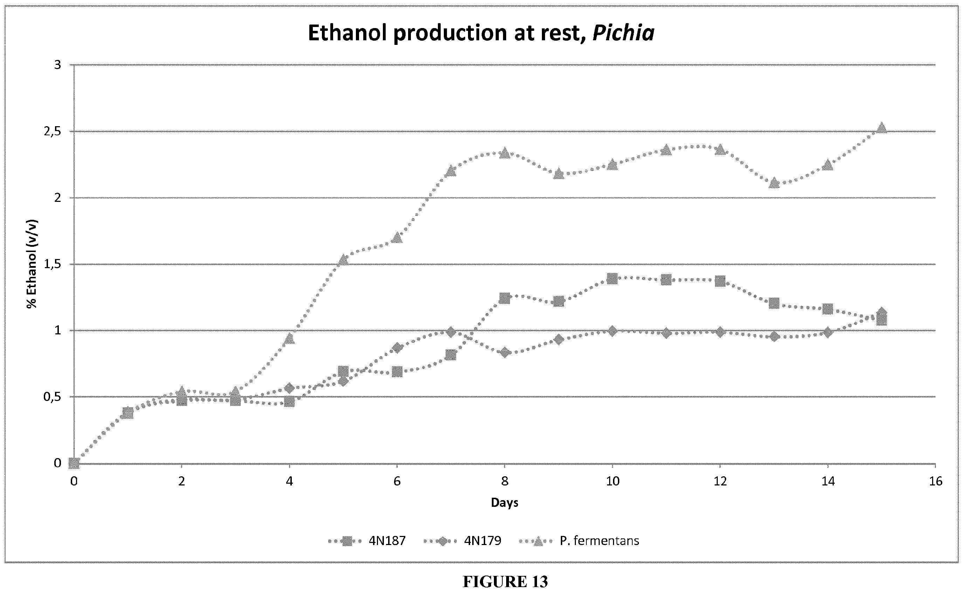

Both strain 4N187 and strain 4N179 follow the same pattern as regards ethanol production (Figure 11). A difference is observed with regard to *P. fermentans* in terms of ethanol production as said strain is able to produce one degree of alcohol more than *P. kluyveri* from day 8 onwards. *P. fermentans* is also the slowest of the three as regards ethanol production, thereby confirming that it is also the slowest when it comes to metabolising reducing sugars. No trend in ethanol metabolism is observed for any of these strains, although minor fluctuations are evident. The lower ethanol production by *P. kluyveri* strains 4N187 and 4N179 with respect to the *P. fermentans* strain is more marked when these yeasts are cultured without stirring (Figure 13), with fermentation using *P. kluyveri* strains giving a very low ethanol concentration of 1% after 7 days but fermentation with the *P. fermentans* strain giving an ethanol concentration of 2.2%.

For all *Saccharomyces* strains, day 4 is defined as the day on which the highest ethanol concentrations are reached. After this point, the *carlsbergensis* and *S. cerevisiae* var. *bayanus* strains exhibit a slight downward trend in these values, possibly due to ethanol metabolism by the yeast as a result of depletion of the sugars previously used as carbon source or evaporation of the ethanol as the process is undertaken with stirring. However, evaporation can be ruled out as no decrease in ethanol concentration is observed for *S. cereviseae,* which is maintained under the same conditions. A higher alcohol content is obtained for *S. cereviseae* and, after day 4, no downward trend is observed in this level, although minor fluctuations that are possibly due to the sampling and analysis procedure may be observed.

### CONCLUSIONS

Strains of the genus *Pichia* are unable to metabolise the sucrose present in orange juice, whereas strains of the genus *Sacharomyces* can metabolise said sugar.

As regards the metabolism of reducing sugars (glucose + fructose), all six strains follow a similar pattern, with concentrations of between 0 and 10 g/L being obtained after 3 days and said sugars being fully depleted after fermentation for 4 days.

Upon depletion of the sugars that can be assimilated by strains of the genus S. *cerevisiae var. bayanus* and *S. carlsbergensis,* the ethanol levels begin to slowly decrease.

Strains of the genus *Pichia* are able to generate ethanol levels of 2.5% after three days and to maintain residual sugars, the majority of which are sucrose (not metabolised) and some residual reducing sugars. Said sugar levels are sufficiently high to avoid the need to add sweeteners.

*P. kluyveri* strains 4N187 and 4N179 provide the best organoleptic properties to the final product and produce lower ethanol concentrations. Although *P. fermentans* behaves in a similar manner to *P. kluyveri* strains 4N187 and 4N179, the final product is similar to that obtained with *Saccharomyces* and does not present optimal organoleptic properties for consumption. Moreover, sucrose must be added to the final product obtained with *P. fermentans* as said strain is able to metabolise sucrose and the ethanol content is higher than for strains 4N187 and 4N179.

## Claims

1. A microorganism from the species *Pichia kluyveri* deposited in the Spanish Type Culture Collection (CECT) with accession number CECT 13055 that is able to ferment glucose and fructose, or a mutant of said microorganism that maintains said ability.

2. A biologically pure culture of a microorganism according to claim 1.

3. A culture medium comprising a microorganism according to claim 1, further comprising 25% (v/v) orange juice and 1% (w/v) of a carbon source, preferably glucose, fructose or mixtures thereof.

4. Use of a microorganism according to claim 1, or a culture according to claim 2, to perform alcoholic fermentation.

5. A growth method for a microorganism according to claim 1, or a culture according to claim 2, comprising inoculation of said microorganism into a culture medium comprising orange juice.

6. A method for obtaining an orange juice by-product comprising inoculation of a microorganism according to claim 1, or a culture according to claim 2, into a culture medium comprising orange juice under conditions that allow fermentation of the reducing sugars in said orange juice.

7. Method according to claim 6, in which fermentation is performed at a temperature of between 15°C and 35°C.

8. Method according to any of claims 6 or 7, in which fermentation is performed for a period of between 1 hour and 20 days.

9. Method according to any of claims 6 to 8, in which fermentation is performed at a temperature of between 20°C and 30°C for a period of between 1 and 5 days.

10. Method according to any of claims 6 to 9, in which fermentation is performed with stirring, recirculation or incorporation of sterile air.

11. Method according to any of claims 6 to 10, in which a decantation of the solids and pasteurisation is performed after fermentation.

12. Method according to any of claims 6 to 11, also comprising a carbonation step.

13. An orange juice by-product comprising a microorganism according to claim 1, with an ethanol concentration of between 0.1% and 3.4% (v/v), preferably 2.5% (v/v), that can be obtained by a method according to any of claims 6 to 12.

14. Product according to claim 13, selected from amongst
- a product according to claim 13 also comprising a compound selected from amongst the group formed by flavouring compounds, acidifiers, dyes, preservatives, antioxidants, emulsifiers, thickeners, stabilisers, compounds for improving the properties of the product of interest for industry and any combination thereof, and
- a product according to claim 13 comprising at least one non-reducing sugar, preferably sucrose.

## Patentansprüche

1. Mikroorganismus von der Spezies *Pichia kluyveri* hinterlegt in der spanischen Kultursammlung (CECT) mit der Zugangsnummer CECT 13055, welcher fähig ist, Glukose und Fruktose zu fermentieren, oder ein Mutant von diesem Mikroorganismus, welcher diese Eigenschaft beibehält.

2. Biologisch reine Kultur von einem Mikroorganismus gemäß Anspruch 1.

3. Kulturmedium umfassend einen Mikroorganismus gemäß Anspruch 1, welches weiterhin 25% (v/v) Orangensaft und 1% (v/v) von einer Kohlenstoffquelle, vorzugsweise Glukose, Fruktose oder Mischungen davon, enthält.

4. Verwendung eines Mikroorganismus gemäß Anspruch 1 oder einer Kultur gemäß Anspruch 2, um eine alkoholische Fermentierung durchzuführen.

5. Wachstumsverfahren für einen Mikroorganismus gemäß Anspruch 1 oder einer Kultur gemäß Anspruch 2, welches Impfen von dem Mikroorganismus in ein Orangensaft-umfassendes Kulturmedium umfasst.

6. Verfahren zum Erhalten eines Nebenprodukts von Orangensaft, welches Impfen eines Mikroorganismus gemäß Anspruch 1 oder einer Kultur gemäß Anspruch 2 in ein Orangensaft-umfassendes Kulturmedium unter Bedingungen, die eine Fermentierung der reduzierenden Zucker in dem Orangensaft erlauben, umfasst.

7. Verfahren gemäß Anspruch 6, in welchem die Fermentierung bei einer Temperatur von zwischen 15°C und 35°C durchgeführt wird.

8. Verfahren gemäß einem der Ansprüche 6 oder 7, in welchem die Fermentierung für eine Dauer von zwischen 1 Stunde und 20 Tagen durchgeführt wird.

9. Verfahren gemäß einem der Ansprüche 6 bis 8, in welchem die Fermentierung bei einer Temperatur von zwischen 20°C und 30°C für eine Dauer von zwischen 1 und 5 Tagen durchgeführt wird.

10. Verfahren gemäß einem der Ansprüche 6 bis 9, in welchem die Fermentierung mit Rühren, Rückzirkulation oder Aufnahme von steriler Luft durchgeführt wird.

11. Verfahren gemäß einem der Ansprüche 6 bis 10, in welchem eine Dekantierung der Feststoffe und Pasteurisierung nach der Fermentierung durchgeführt werden.

12. Verfahren gemäß einem der Ansprüche 6 bis 11 ebenfalls umfassend einen Karbonisierungsschritt.

13. Nebenprodukt von Orangensaft, welches einen Mikroorganismus gemäß Anspruch 1 umfasst, mit einer Ethanolkonzentration von zwischen 0,1% und 3,4% (v/v), vorzugsweise 2,5% (v/v), welches durch ein Verfahren gemäß einem der Ansprüche 6 bis 12 erhalten werden kann.

14. Produkt gemäß Anspruch 13 ausgewählt aus
- einem Produkt gemäß Anspruch 13 ebenfalls umfassend eine Verbindung ausgewählt aus der Gruppe, welche durch Aromastoffe, Säuerungsmittel, Farbstoffe, Konservierungsmittel, Antioxidationsmittel, Emulgatoren, Verdickungsmittel, Stabilisationsmittel, Verbindungen zur Verbesserung der für die Industrie relevanten Eigenschaften des Produkts und jeder Kombination daraus gebildet wird, und
- ein Produkt gemäß Anspruch 13 umfassend mindestens einen nichtreduzierenden Zucker, bevorzugt Sucrose.

## Revendications

1. Un microorganisme de l'espèce *Pichia kluyveri* déposé dans la Collection Espagnole de Cultures Types (CECT) avec le numéro d'accès CECT 13055 qui est apte à fermenter le glucose et le fructose, ou un mutant dudit microorganisme qui maintient ladite aptitude.

2. Une culture biologiquement pure d'un microorganisme selon la revendication 1.

3. Un milieu de culture comprenant un microorganisme selon la revendication 1, comprenant en outre 25% (v/v) de jus d'orange et 1% (p/v) d'une source de carbone, de préférence du glucose, du fructose ou des mélanges de ceux-ci.

4. Utilisation d'un microorganisme selon la revendication 1, ou d'une culture selon la revendication 2, pour effectuer une fermentation alcoolique.

5. Un procédé de croissance pour un microorganisme selon la revendication 1, ou pour une culture selon la revendication 2, comprenant l'inoculation dudit microorganisme dans un milieu de culture comprenant du jus d'orange.

6. Un procédé pour obtenir un produit dérivé de jus d'orange comprenant l'inoculation d'un microorganisme selon la revendication 1 ou d'une culture selon la revendication 2 dans un milieu de culture comprenant du jus d'orange dans des conditions permettant la fermentation des sucres réducteurs dans ledit jus d'orange.

7. Procédé selon la revendication 6, dans lequel la fermentation est effectuée à une température comprise entre 15°C et 35°C.

8. Procédé selon l'une quelconque des revendications 6 ou 7, dans lequel la fermentation est effectuée pendant une durée comprise entre 1 heure et 20 jours.

9. Procédé selon l'une quelconque des revendications 6 à 8, dans lequel la fermentation est effectuée à une température comprise entre 20°C et 30°C pendant une période comprise entre 1 et 5 jours.

10. Procédé selon l'une quelconque des revendications 6 à 9, dans lequel la fermentation est effectuée avec agitation, recirculation ou incorporation d'air stérile.

11. Procédé selon l'une quelconque des revendications 6 à 10, dans lequel une décantation des solides et une pasteurisation est effectuée après la fermentation.

12. Procédé selon l'une quelconque des revendications 6 à 11, comprenant également une étape de carbonatation.

13. Un produit dérivé de jus d'orange comprenant un microorganisme selon la revendication 1, avec une concentration en éthanol comprise entre 0,1% et 3,4% (v/v), de préférence de 2,5% (v/v), qui peut être obtenue par un procédé selon l'une quelconque des revendications 6 à 12.

14. Produit selon la revendication 13, choisi parmi :
- un produit selon la revendication 13, comprenant également un composé choisi parmi le groupe formé par: des composés aromatisants, des acidifiants, des colorants, des conservateurs, des antioxydants, des émulsifiants, des épaississants, des stabilisants, des composés pour améliorer les propriétés du produit d'intérêt pour l'industrie, et toute combinaison de ceux-ci, et
- un produit selon la revendication 13 comprenant au moins un sucre non réducteur, de préférence du saccharose.
